# FASCICULE DE BREVET EUROPEEN

(11) **EP 2 739 603 B1**
(45) Date de publication et mention de la délivrance du brevet: **17.06.2015**
(21) Numéro de dépôt: 12744111.1
(22) Date de dépôt: 26.07.2012
(51) Int. Cl.: C07C 227/04, C07C 229/08

(54) **PROCEDE DE SYNTHESE D'UN OMEGA-AMINOACIDE OU ESTER A PARTIR D'UN ACIDE OU ESTER GRAS MONO-INSATURE**
VERFAHREN ZUR SYNTHESE EINER OMEGA-AMINOSÄURE ODER EINES ESTERS AUS EINER EINFACH GESÄTTIGTEN FETTSÄURE ODER EINEM ESTER
METHOD FOR SYNTHESIZING AN OMEGA-AMINO ACID OR ESTER FROM A MONOUNSATURATED FATTY ACID OR ESTER

(30) Priorité: 01.08.2011 FR 1157020
(43) Date de publication de la demande: 11.06.2014
(73) Titulaire: Arkema France, 92700 Colombes (FR)
(72) Inventeur: COUTURIER, Jean-Luc, F-69006 Lyon (FR); DUBOIS, Jean-Luc, F-69390 Millery (FR)
(74) Mandataire: Hérard, Elise
(86) Numéro de dépôt international: PCT/FR2012/051770
(87) Numéro de publication internationale: WO 2013/017782

(56) Documents cités:
- WO-A1-2010/055273
- MUDASSAR ABBAS ET AL: "As low as reasonably achievable catalyst loadings in the cross metathesis of olefins with ethyl acrylate", TETRAHEDRON LETTERS, vol. 52, no. 20, 1 mai 2011 (2011-05-01), pages 2560-2562, XP055010903, ISSN: 0040-4039, DOI: 10.1016/j.tetlet.2011.03.038

## Description

Le travail qui a conduit à cette invention a reçu un financement de la part de l'Union Européenne dans le cadre du 7ième Programme Cadre (FP7/2007-2013) sous le numéro de projet N°241718 EUROBIOREF.

L'invention vise un procédé de synthèse d'acides ω-amino-alcanoïques ou de leurs esters à partir d'acides gras naturels insaturés transitant par un composé intermédiaire de type nitrile ω-insaturé.

L'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amide -CO-NH-, C'est ainsi que sont connus les Nylon-6, Nylon 6-6, Nylon 6-10, Nylon 7, Nylon 8, Nylon 9, Nylon 11, Nylon 13, etc...

Ces monomères sont par exemple fabriqués par voie de synthèse chimique en utilisant notamment comme matière première des oléfines en C2 à C4, des cycloalcanes ou du benzène, mais aussi de l'huile de ricin (Nylon 11), de l'huile érucique ou lesquérolique (Nylon 13)...

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan 11^{®}. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part l'undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.

Les principaux travaux ont porté sur la synthèse de l'acide amino-9-nonanoïque qui est le précurseur du Nylon 9 à partir de l'acide oléique d'origine naturelle. En ce qui concerne ce monomère particulier on peut citer *l'ouvrage* « n-Nylons, Their Synthesis, Structure and Properties » - 1997 Ed.J. Wiley et Sons dont le chapitre 2.9 (pages 381 à 389) est consacré au 9-Nylon. Cet article donne la synthèse des réalisations et des travaux conduits sur le sujet. On y mentionne, page 381, le procédé développé par l'ex Union Soviétique ayant conduit à la commercialisation du Pelargon®. On y mentionne, page 384, également un procédé développé au Japon utilisant l'acide oléique venant de l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.

Pour être complet sur l'état de l'art en la matière, il faut citer les nombreux articles publiés par E. H. Pryde et al. entre 1962 et 1975 dans - Journal of the American Oil Chemists ' Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et R.B. Perkins et al. « Nylon-9 from Unsaturated Fatty Derivatives : Préparation and Characterization » JAOCS, Vol. 52 pages 473-477. Il est à noter que le premier de ces articles fait aussi référence page 498 à des travaux antérieurs réalisés par des Japonais : H. Otsuki et H. Funahashi.

Pour résumer cette partie de l'état de l'art visant ce type de synthèse du « Nylon 9 » à partir d'huiles végétales on peut décrire le mécanisme réactionnel simplifié suivant appliqué à l'ester oléique, extrait des huiles par méthanolyse :
Ozonolyse réductrice

   H₃C-(CH₂)₇-CH=CH-(CH₂)₇-COOCH₃ + (O₃, H₂) → HOC-(CH₂)₇-COOCH₃ + H₃C-(CH₂)₇-COH
Amination réductrice

   HOC-(CH₂)₇-COOCH₃ + (NH₃, H₂) → H₂N-(CH₂)₈-COOCH₃ + H₂O
Hydrolyse

   H₂N-(CH₂)₈-COOCH₃ +H₂O → H₂N-(CH₂)₈-COOH + CH₃OH

Cette voie très séduisante sur le plan réactionnel présente cependant un inconvénient économique important constitué par la production lors de la première étape d'un aldéhyde à longue chaîne (9 atomes de carbone au total) pratiquement pas valorisable notamment dans l'industrie des polymères de type polyamides.

Le brevet UK n° 741,739 décrit pour sa part la synthèse de ce même acide à partir de l'acide oléique mais en utilisant la voie oléonitrile. Le schéma réactionnel simplifié de ce procédé est le suivant. Une voie analogue est citée dans l'article de R.B. Perkins et al cité ci-dessus p. 475.

H₃C-(CH₂)₇-CH=CH-(CH₂)₇-COOH + NH₃ → H₃C-(CH₂)₇-CH=CH-(CH₂)₇-CN + 2 H₂O

H₃C-(CH₂)₇-CH=CH-(CH₂)₇-CN + (O₃ + H₂O) → H₃C-(CH₂)₇-COOH + CN-(CH₂)₇-COOH

CN-(CH₂)₇-COOH + 2 H₂ → H₂N-(CH₂)₈-COOH

Cette synthèse conduit à l'acide pélargonique H₃C-(CH₂)₇-COOH comme sous-produit.

WO 2010/055273 divulgue un procédé de synthèse d'un ω-aminoacide(ester) de formule ROOC-(CH2)_{q}-CH₂NH₂ dans laquelle R est H ou un radical alkyle comportant de 1 à 4 atomes de carbone et q un indice entier compris entre 2 et 13 à partir d'un acide(ester) gras mono-insaturé. Ce procédé comporte une étape réactionnelle d'ammoniation avec un premier stade où l'acide(ester) gras mono-insaturé de départ est transformé en un nitrile ω-insaturé en deux étapes successives (d'ordre indifférent) éthénolyse et ammoniation. Dans un deuxième stade, le nitrile ω-insaturé est transformé en nitrile acide/ester en C₁-C₄ par réaction de métathèse croisée avec un acrylate de C₁-C₄-alkyle. Dans un dernier stade, une réaction d'hydrogénation conduit à l'w-aminoacide(ester) de formule ROOC-(CH₂)_{q}-CH₂NH₂.

Dans l'article "As low as reasonably achievable catalyst loadings in the cross metathesis of olefins with ethyl acrylate", Tetrahedron Letters, Vol. 52(20) pages 2560-2562, M. Abbas et C. Slugovc ont étudié l'aptitude d'une série de catalyseurs au ruthénium pour la métathèse d'oléfines dans la métathèse croisée du 1,9-décadiène avec l'acrylate d'éthyle.

La présente invention vise à proposer un nouveau procédé pour synthétiser toute une gamme d'acides ω-amino-alcanoïques ou de leurs esters à partir d'acides gras naturels insaturés.

Le problème est donc de trouver un procédé de synthèse de divers ω-amino-acides de formule H₂N-(CH₂)ₙ-COOH (et de leurs polymères) dans laquelle n est compris entre 3 et 14 à partir de matières premières renouvelables (de très large accès et donc peu onéreuses), simple à mettre en oeuvre tout en évitant d'une part les contraintes environnementales évoquées précédemment et d'autre part les handicaps économiques dus aux sous-produits des réactions.

La solution proposée consiste à travailler à partir de matières premières constituées par des acides gras insaturés à longue chaîne naturels, à les transformer dans un premier stade en nitriles ω-insaturés puis ensuite dans un deuxième stade à « réinsérer » une fonction acide carboxylique dans le composé par action sur la double liaison terminale du nitrile ω-insaturé, par réaction de métathèse croisée avec un composé de type acrylate.

On entend par acide gras naturel un acide issu des milieux végétal ou animal, y compris les algues, plus généralement du règne végétal, et donc renouvelable. Cet acide comportera au moins une insaturation oléfinique dont la localisation en position x par rapport au groupement acide (delta x) et comportant au moins 10 et de préférence au moins 14 atomes de carbone par molécule permettra de déterminer la formule de l'ω-amino-acide final.

On peut citer à titre d'exemples de tels acides, les acides en C10, les acides obtusilique (cis-4-décénoïque) et caproléique (cis-9-décénoïque), les acides en C12, les acides lauroléique (cis-9-dodécènoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque).

Ces divers acides sont issus des huiles végétales extraites de diverses plantes oléagineuses telles que le tournesol, le colza, le ricin, le lesquerella, l'olive, le soja, le palmier, l'avocat, l'argousier, la coriandre, le céleri, l'aneth, la carotte, le fenouil, la cameline, le Limnanthes Alba (meadowfoam).

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise un procédé de synthèse d'un ω-aminoacide(ester) de formule R₃OOC-(CH₂)_{q}-CH₂NH₂ dans laquelle R₃ est H ou un radical n-butyle et q un indice entier compris entre 2 et 13, à partir d'un acide(ester) gras mono-insaturé de formule (R₁-CH=CH-(CH₂)ₚ-COO)ₓR₂ dans laquelle x représente 1, 2 ou 3, R₁ est H ou un radical hydrocarboné comprenant de 4 à 11 atomes de carbone et le cas échéant une fonction hydroxyle, R₂ est H ou un radical alkyle comportant de 2 à 4 atomes de carbone et pouvant contenir un ou plusieurs hétérotatomes et p un indice entier compris entre 2 et 11 , comportant une étape réactionnelle d'ammoniation conduisant à la transformation de la fonction carbonyle en fonction nitrile caractérisé en ce que
- dans un premier stade on transforme l'acide/ester gras insaturé en un nitrile ω-insaturé de formule CH₂=CH-(CH₂)ₚ-CN en deux étapes successives (d'ordre indifférent) éthénolyse et ammoniation puis,
- dans un deuxième stade on transforme ce nitrile ω-insaturé en un nitrile ester de formule R₃OOC- CH=CH-(CH₂)ₚ-CN dans laquelle R₃ est n-butyle, par une réaction de métathèse croisée du nitrile ω-insaturé avec un acrylate de formule CH₂=CH-COOR₃ avec un catalyseur de formule (I), 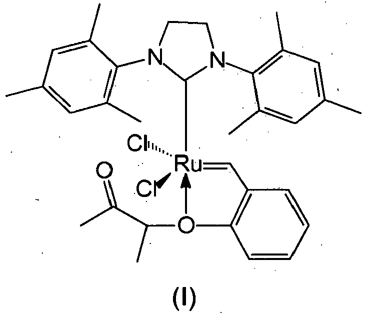 puis,
- dans un troisième stade on hydrogène le nitrile ester en ω-aminoacide(ester) de formule R₃OOC-(CH₂)_{q}-CH₂NH₂.

Le processus réactionnel est alors le suivant.
Premier stade :

R₁-CH=CH-(CH₂)ₚ-COOH + CH₂=CH₂ ⇔ CH₂=CH-(CH₂)ₚ-COOH +CH2=CH-R₁

CH₂=CH-(CH₂)ₚ-COOH + NH₃ → CH₂=CH-(CH₂)ₚ-CN + 2 H₂O

ou en inversant l'ordre des réactions

R₁-CH=CH-(CH₂)ₚ-COOH + NH₃ → R₁-CH=CH-(CH₂)ₚ-CN +2 H₂O

R₁-CH=CH-(CH₂)ₚ-CN + CH₂=CH₂ ⇔ CH₂=CH-(CH₂)ₚ-CN + CH₂=CH-R₁

Deuxième stade :

CH₂=CH-(CH₂)ₚ-CN + CH₂=CH-COOR₃ ⇔ R₃OOC-CH=CH-(CH₂)ₚ-CN + CH₂=CH₂

Troisième stade :

R₃OOC-CH=CH-(CH₂)ₚ-CN + 3 H₂ → R₃OOC-(CH₂)_{q}-CH₂NH₂.

Dans ce mode réalisation du procédé q est égal à p+2.

Appliqué à l'acide oléique le processus devient
Premier stade :

CH₃-(CH₂)₇-CH=CH-(CH₂)₇-COOH + CH₂=CH₂ ⇔ CH₂=CH-(CH₂)₇-COOH + CH₂=CH-(CH₂)₇-CH₃

CH₂=CH-(CH₂)₇-COOH + NH₃ → CH₂=CH-(CH₂)₇-CN + 2 H₂O

ou en inversant l'ordre des réactions

CH₃-(CH₂)₇-CH=CH-(CH₂)₇-COOH + NH₃ → CH₃-(CH₂)₇-CH=CH-(CH₂)₇-CN +2 H₂O

CH₃-(CH₂)₇-CH=CH-(CH₂)₇-CN + CH₂=CH₂ ⇔ CH₂=CH-(CH₂)₇-CN + CH₂=CH-(CH₂)₇-CH₃

Deuxième stade :

CH₂=CH-(CH₂)₇-CN + CH₂=CH-COOR3 ⇔ R₃OOC-CH=CH-(CH₂)₇ -CN + CH₂=CH₂

Troisième stade :
seconde variante : R₃OOC-CH=CH-(CH₂)₇-CN + 3 H₂→ R₃OOC-(CH₂)₉-CH₂NH₂

Les seuls « sous-produits » formés sont une α-oléfine à longue chaîne, comportant le cas échéant une fonction hydroxyle.

Dans une variante simplifiée de réalisation du procédé de l'invention on peut gagner une étape en synthétisant lors du premier stade le nitrile de l'acide/ester gras de formule R₁-CH=CH-(CH₂)ₚ-CN par ammoniation de l'acide/ester d'origine puis en soumettant ce dernier à une métathèse croisée avec un acrylate R₃OOC-CH=CH₂ pour obtenir le nitrile-ester de formule R₃OOC-CH=CH-(CH₂)ₚ-CN qui sera ensuite hydrogéné en R₃OOC-(CH₂)ₚ₊₂-CH₂NH₂.

Dans une autre variante du procédé utilisant comme matière première les acides gras hydroxylés tels que l'acide ricirioléique et l'acide lesquérolique qui répondent à la formule générale R₁-CH=CH-(CH₂)ₚ-COOH avec R₁ égal à CH₃-(CH₂)₅CHOH-CH₂- et p égal respectivement 7 et 9 on.soumet l'acide sous sa forme ester méthylique à une pyrolyse conduisant à un ester ω-insaturé de formule CH₂=CH-(CH₂)ₚ₊₁-COOCH₃ qui est transformé, directement ou en passant par l'acide, en un nitrile ω-insaturé de même nature que celui du composé intermédiaire obtenu au terme du premier stade du procédé décrit ci-dessus. Cette variante consiste donc à remplacer pour ces acides gras particuliers l'éthénolyse initiale par une pyrolyse.

Les stades suivants du procédé sont analogues à ceux du procédé décrit ci-dessus. Ils conduisent donc à des composés de formules R₃OOC-(CH₂)_{q}-CH₂NH₂ dans laquelle q est égal à p+3.

Ainsi, dans des modes de réalisations préférés de l'invention :
- lors du premier stade on effectue tout d'abord l'éthénolyse de l'acide (ester) pour la faire suivre par l'ammoniation de l'acide ω-alcénoïque ;
- lors du premier stade on effectue tout d'abord l'ammoniation de l'acide (ester) pour la faire suivre par l'éthénolyse du nitrile de l'acide gras de départ ;
- lors du premier stade on effectue tout d'abord la pyrolyse de l'acide (ester) gras hydroxylé pour la faire suivre par l'ammoniation de l'acide(ester) ω-alcénoïque issu de la pyrolyse ;
- lors du premier stade on effectue l'ammoniation de l'acide (ester) sans procéder à la réaction d'éthénolyse ;
- lors du deuxième stade on soumet le produit issu du premier stade à une réaction de métathèse croisée avec le composé de type acrylate ;
- le composé issu du deuxième stade est soumis à une hydrogénation.

Dans un mode de réalisation particulier de l'invention, dans la formule (R₁-CH=CH-(CH₂)ₚ-COO)ₓR₂, x représente 3 et le radical R2 est CH₂-CH-CH₂, ou x représente 2 et R₂ est CH₂-CH-CH₂OH ou CH₂-CHOH-CH₂.

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru Topics in Catalysis of unsaturated fatty esters and oil Vol. 27, Nos. 1-4, February 2004 (Plenum Publishing).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que Rhénium, Molybdène et Tungstène déposés sur alumine ou silice.

Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

La réaction de métathèse croisée avec l'éthylène lors de l'une des étapes de la première phase peut être réalisée avec tout catalyseur de métathèse actif et sélectif, et de préférence est conduite à une température comprise entre 20 et 100°C à une pression de 1 à 30 bars en présence d'un catalyseur de métathèse classique par exemple de type Ruthénium. Le temps de réaction est choisi en fonction des réactifs mis en oeuvre et pour atteindre au plus près l'équilibre de la réaction. La réaction est effectuée sous une pression d'éthylène. Elle peut être effectuée directement sur l'huile, l'ester, et sur l'acide gras.

Les catalyseurs au ruthénium sont choisis de préférence parmi les catalyseurs chargés ou non-chargés de formule générale :

(X1)a (X2)bRu(carbène C) (L1)c(L2)d

dans laquelle :
- a, b, c, d sont des nombres entiers avec a et b égaux à 0, 1 ou 2 ; c et d égaux à 0, 1, 2, 3 ou 4;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, le tétraphénylborate et dérivés. X1 ou X2 peuvent être liés à Y1 ou Y2 ou au (carbène C) de façon à former un ligand bidenté (ou chélate) sur le ruthénium ; et
- L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique L1 ou L2 peuvent être liés au "carbène C " de façon à former un ligand bidenté ou chélate,

Le "carbène C" pourra être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHR ou allénylidènes Ru=C=C=CR1R2 ou indénylidènes.

Un groupe fonctionnel permettant d'améliorer la rétention du complexe du ruthénium dans le liquide ionique peut être greffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

La réaction de métathèse croisée avec l'acrylate de butyle est réalisée dans des conditions parfaitement connues. La température de réaction est comprise entre 20 et 100 °C généralement à la pression atmosphérique pour permettre un dégagement aisé de l'éthylène en présence d'un catalyseur à base de ruthénium, de formule (I) donnée ci-dessus.

Le schéma réactionnel de la synthèse des nitriles à partir des acides, bien connue de l'homme de l'art, peut se résumer de la façon suivante.

R-COOH + NH_{3 →} [R-COO-NH₄⁺] → [R-CONH₂] + H₂O → RCN + H₂O

Ce schéma s'applique tout aussi bien aux acides (esters) gras naturels qu'aux acides gras ω-insaturés.

Le procédé peut être conduit en batch en phase liquide ou gazeuse ou en continu en phase gazeuse. La réaction est conduite à température élevée > 250 °C et présence d'un catalyseur qui est généralement un oxyde métallique et le plus fréquemment l'oxyde de zinc. L'élimination en continu dé l'eau formée en entraînant de surcroît l'ammoniac qui n'a pas réagi permet un achèvement rapide de la réaction.

La réaction de pyrolyse mise en oeuvre dans la variante du procédé est réalisée sur la forme ester de l'acide gras hydroxylé concerné en général l'ester méthylique. La réaction est conduite à haute température, comprise entre 400 et 750 °C et de préférence entre 500 et 600 °C en présence de vapeur d'eau surchauffée.

La réaction de pyrolyse appliquée au ricinoléate de méthyle répond au processus suivant :

CH₃-(CH₂)₅CHOH-CH₂-CH=CH-(CH₂)₇-COOCH₃ + Δ → CH₃-(CH₂)₅CHO + CH₂=CH-(CH₂)₈-COOCH₃

Elle est suivie par une ammoniation :

CH₂=CH-(CH₂)₈-COOCH₃ + NH₃ → CH₂=CH-(CH₂)₈-CN + 2 H₂O.

L'étape de synthèse des ω-aminoacides(esters) gras à partir des nitrile-esters gras consiste en une hydrogénation classique. Les catalyseurs sont nombreux mais préférentiellement on utilise les Nickel et Cobalt de Raney. Afin de favoriser la formation de l'amine primaire, on opère avec une pression partielle d'ammoniac. Enfin, la réduction de la fonction nitrile en amine primaire est bien connue de l'homme de l'art.

Dans le procédé de l'invention, on peut traiter l'acide gras soit sous sa forme acide, soit sous sa forme ester, y compris triglycéride ou diglycéride. Le passage d'une forme à l'autre, par méthanolyse, estérification ou hydrolyse, parfaitement banal ne constitue pas une transformation chimique au sens du procédé.

Tous les mécanismes détaillés ci-dessous illustrent, pour faciliter l'exposé, la synthèse des acides. Cependant, la métathèse est aussi efficace avec un ester et même plus efficace, le milieu étant généralement plus anhydre. De la même manière, les schémas illustrent des réactions avec l'isomère trans des acides (ou esters) ; les mécanismes sont aussi bien applicables aux isomères cis.

Le mécanisme réactionnel de cette réaction est illustrée par le schéma 1 ci-dessous.

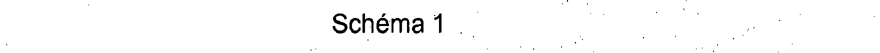

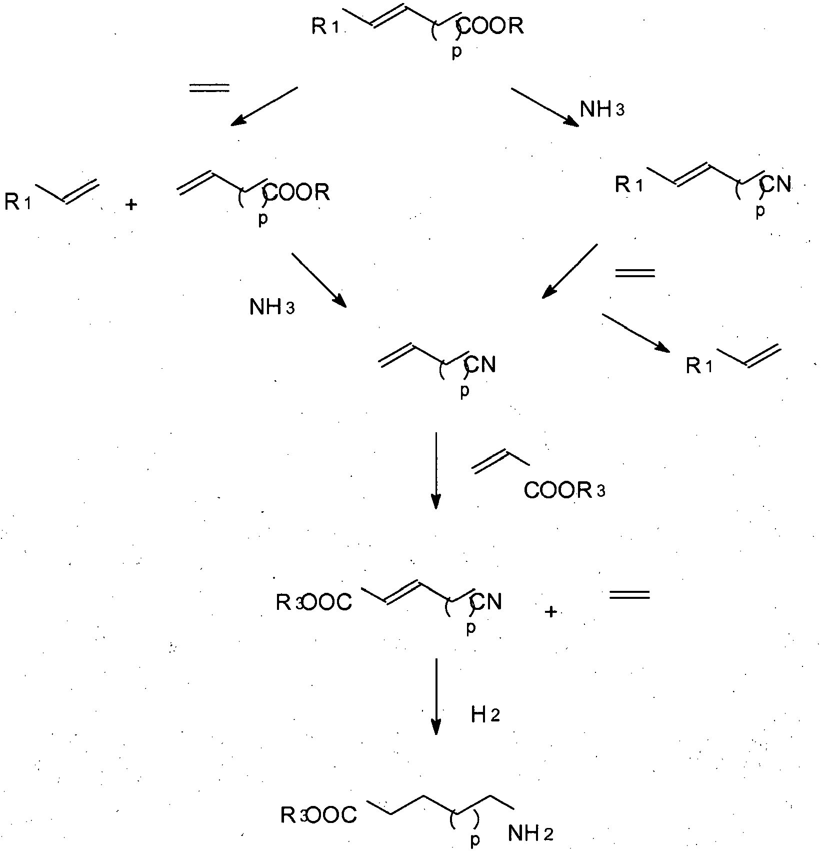

Dans le schéma ci-dessus q=p+2.

La variante de mise en oeuvre du procédé de l'invention appliquée aux acides gras insaturés hydroxylés est illustrée par le schéma 2 ci-dessous.

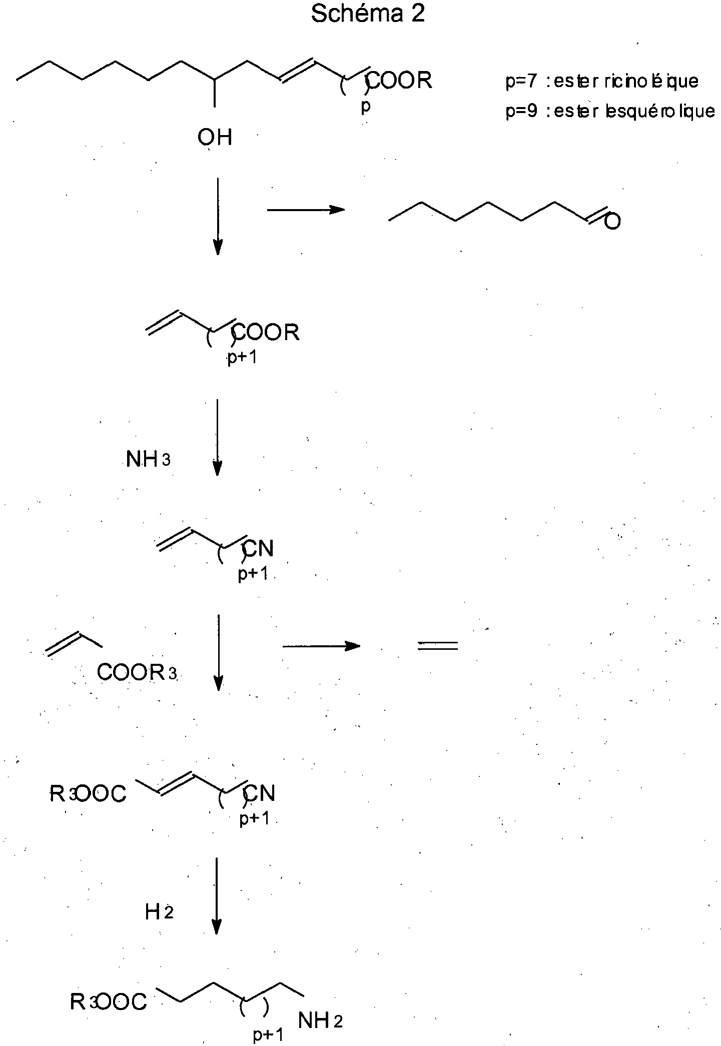

Ce document concerne en outre l'aminoacide ou aminoester d'origine renouvelable de formule générale NH₂CH₂-(CH₂)_{q}-COOR, R étant soit H, soit un radical butyle.

Par aminoacides ou aminoesters d'origine renouvelable, on entend les aminoacides ou aminoesters qui comprennent du carbone d'origine renouvelable.

En mettant en oeuvre le procédé de l'invention, il sera possible de synthétiser toute une gamme d'ω-aminoacides.

L'acide 4-aminobutanoïque est obtenu à partir de l'acide eicosénoïque.

L'acide 6-aminohexanoïque est obtenu à partir des acides obtusilique, lindérique et tsuzuique.

L'acide 7-aminoheptanoïque est obtenu à partir de l'acide physétérique.

L'acide 8-aminooctanoïque est obtenu à partir de l'acide petrosélinique.

L'acide 11-aminoundécylénique est obtenu à partir des acides lauroléïque, caproléique, myristoléique, palmitoléique, oléique, élaidique, ricinoléique, et gadoléique.

L'acide 12-aminododécylénique est obtenu à partir des acides ricinoléique et lesquérolique.

L'acide 13-aminotridécylénique est obtenu à partir des acides vaccénique, gondoïque, cétoléique et lesquérolique.

L'acide 15-aminopentadécylénique est obtenu à partir de l'acide érucique.

L'invention est illustrée par les exemples suivants.

### Exemple 1

Cet exemple illustre la première étape d'éthénolyse de l'oléate de méthyle selon le procédé objet de l'invention. On utilise pour cette réaction le catalyseur complexe [RuCl₂(=CHPh)(IMesH₂)(PCy₃)] dont la formule (A) est donnée ci-dessous. On effectue la réaction dans CH₂Cl₂, à une concentration de 0,05 M d'oléate de méthyle et 0,2 M d'éthylène à une température de 55°C à la pression atmosphérique, et pendant 6 heures, en présence du catalyseur à une concentration de 5 % molaire par rapport à l'oléate de méthyle. Les rendements sont déterminés par analyse chromatographique. On peut mesurer un rendement en 9-décénoate de méthyle CH₂=CH-(CH₂)₇-COOCH₃ et en 1-décène de 55 % molaire.

### Catalyseur de formule (A)

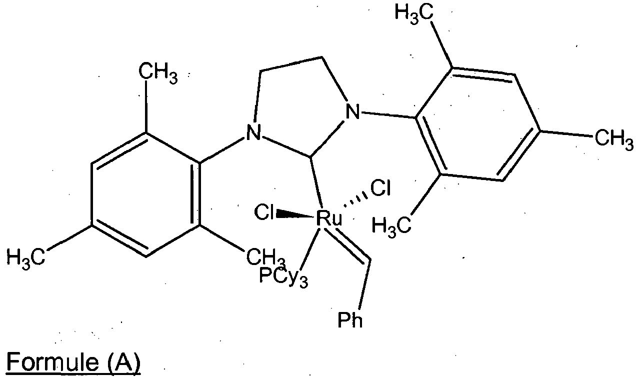

### Exemple 2

Cet exemple illustre la deuxième étape d'ammoniation transformant l'acide 9-décénoïque, issu après hydrolyse de l'ester de la première étape, en nitrile de formule CN-(CH₂)₇- CH=CH₂.

La réaction d'ammoniation de l'acide 9-décénoïque (3,5g) pour former le nitrile ω-insaturé de formule CN-(CH₂)₇-CH=CH₂ est conduite en batch avec introduction d'ammoniac en excès molaire par rapport à l'acide et à une température de 300°C à la pression atmosphérique (en phase gazeuse), en présence d'un catalyseur d'oxyde de zinc. Le réacteur est équipé d'un condenseur à 100 °C. De l'ammoniac est aussi injectée en continu pendant 6 heures. L'élimination en continu de l'eau formée entraîne l'ammoniac en excès permet un achèvement rapide de la réaction. On récupère 2,6 g du nitrile qui sont séparés par distillation sous vide.

### Exemple 3 (comparatif)

Cet exemple illustre une réaction de métathèse croisée entre un nitrile de formule CN-(CH₂)₇-CH=CH₂ issu de l'étape de l'exemple 2 avec l'acrylate de méthyle selon la réaction :

CH₂=CH-(CH₂)₇-CN + CH₂=CH-COOCH₃ ⇔ CH₃OOC-CH=CH-(CH₂)₇ -CN + CH₂=CH₂

Dans un tube de Schlenk de 50 ml, on charge 83 mg de 9-décènenitrile (0,55 mmol), 86 mg d'acrylate de méthyle (1 mmol) et 10 ml de toluène distillé sur sodium-benzophénone. On rajoute 1,5 mg (2,4.10⁻³ mmol) de catalyseur de Hoveyda-Grubbs de seconde génération [(1,3-bis(2,4,6-triméthylphényl)-2-imidazolidinylidène)dichloro(o-isopropoxyphénylméthylène] ruthénium, commercialisé par la société Aldrich®). Sous azote et sous agitation magnétique, on chauffe à 100°C et on laisse réagir 1 heure. Le mélange réactionnel est analysé par chromatographie en phase gaz (étalon dodécane). La conversion est de 70%. La sélectivité en nitrile ester de méthyle (mélange cis + trans) est de 100%.

### Exemple 4

Cet exemple illustre la variante avec inversion de l'ordre des 2 étapes de la phase 1: ammoniation de l'acide gras insaturé puis éthénolyse du nitrile insaturé.

L'ammoniation l'acide oléique est conduite en batch avec introduction d'ammoniac en excès molaire par rapport à l'acide et à une température de 300°C à la pression atmosphérique (en phase gazeuse), en présence d'un catalyseur d'oxyde de zinc. L'élimination en continu de l'eau formée entraîne l'ammoniac en excès permet un achèvement rapide de la réaction.

L'éthénolyse du nitrile de l'acide oléique est conduite à 60°C à la pression atmosphérique en présence d'un catalyseur à base de Ruthénium [RuCl₂(=CHPh)(IMeSH₂)(PCy₃)] en utilisant un excès d'éthylène pour obtenir l'acide 9-décénoïque CH₂=CH-(CH₂)₇-COOH. Les rendements sont déterminés par analyse chromatographique. Au terme de la réaction, 6 heures, on sépare l'α-oléfine en C10 par distillation sous vide pour obtenir le g-décènenitrile CH₂=CH-(CH₂)₇-CN. Les rendements sont déterminés par analyse chromatographique. On peut mesurer un rendement de 55 %.

### Exemple 5

Pyrolyse d'acide gras hydroxylé.

Le triglycéride de l'acide ricinoléique est transestérifié par un excès de méthanol en présence de méthylate de sodium.

L'ester est alors vaporisé à 225 °C et ensuite mélangé avec de la vapeur d'eau surchauffée (620°C). La réaction est brève, une dizaine de secondes environ. On procède ensuite à la purification de l'undécénoate de méthyle, tout d'abord par refroidissement du milieu qui permet l'extraction de l'eau puis par une série de distillations permettant la séparation de l'ester et des sous-produits de la réaction.

### Exemple 6 : métathèse croisée 10-undécènenitrile-acrylate de butyle

Le 10-undécènenitrile est préalablement purifié sur alumine (alumine basique VWR Normapur). 10g d'alumine sont chargés dans une colonne et 20g de 10-undécènenitrile sont percolés sur la colonne à pression atmosphérique.

Le réacteur de métathèse est un réacteur en verre double-enveloppe de 250ml muni d'une agitation mécanique, d'une réfrigérant, d'une sonde de température, d'une arrivée d'azote et d'un pousse-seringue pour additionner le catalyseur de métathèse en continu.

Dans un le réacteur purgé à l'azote, on charge 5g de 10-undécènenitrile (30 mmol), 7,6g d'acrylate de butyle (60 mmol) et 50g de toluène séché sur tamis moléculaire. On charge dans une seringue 0,5 mg de catalyseur de formule (I) donnée ci-dessus (catalyseur fourni par la société UMICORE - 7,5.10-4 mmol-0,0025% mol par rapport au 10-undécènenitrile) dissous dans 5ml de toluène. On chauffe à 100°C puis on ajoute le catalyseur via le pousse seringue sur une période de 3 heures. Le mélange réactionnel résultant est analysé par chromatographie en phase gaz pour déterminer la conversion du 10-undécènenitrile et les sélectivités en nitrile-ester C12 insaturé (produit de métathèse croisée) et en dinitrile C20 insaturé (produit de self-métathèse).

### L'exemple 7 (comparatif)

L'exemple 7 est réalisé dans les mêmes conditions que l'exemple 6 mais en utilisant le catalyseur de formule (II) ci-dessous à même quantité molaire.

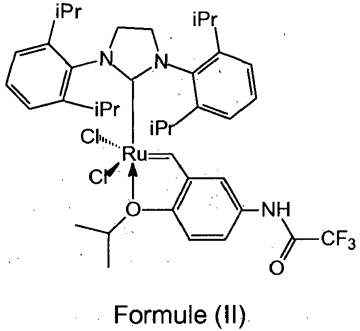

### L'exemple 8 (comparatif)

L'exemple 8 est réalisé dans les mêmes conditions que l'exemple 6 mais en utilisant le catalyseur de formule (III) ci-dessous à même quantité molaire.

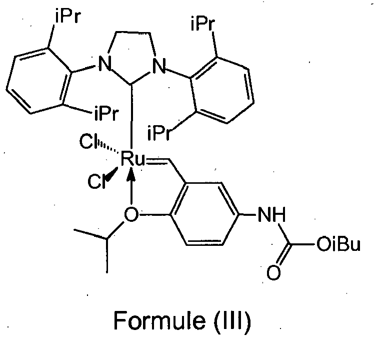

### L'exemple 9 (comparatif)

L'exemple 9 est réalisé dans les mêmes conditions que l'exemple 6 mais en remplaçant l'acrylate de butyle par l'acrylate de méthyle à même quantité molaire.

Tous les résultats des exemples 6 à 9 sont résumés dans le tableau 1 suivant.

**Tableau 1**

| Exemple | Acrylate | Catalyseur | Conversion (%) | Sélectivité (%) Nitrile-ester | Sélectivité (%) |
|---|---|---|---|---|---|
| | | | Nitrile C₁₁ | C₁₂ | Dinitrile C₂₀ |
| 6 | ABu | formule (I) | 63 | 66 | 34 |
| 7 | ABu | Formule (II) | 34 | 25 | 75 |
| 8 | ABu | Formule (III) | 21 | 26 | 74 |
| 9 | AMe | Formule (I) | 10 | 30 | 70 |

Ces résultats montrent que seul le couple ABu/catalyseur de formule (I) permet d'obtenir des bonnes conversions et sélectivités en métathèse croisée à basse teneur en catalyseur.

## Revendications

1. Procédé de synthèse d'un ω-aminoacide(ester) de formule R₃OOO(CH₂)_{q}-CH₂NH₂ dans laquelle R₃ est H ou un radical n-butyle et q un indice entier compris entre 2 et 13, à partir d'un acide(ester) gras mono-insaturé de formule (R₁-CH=CH-(CH₂)ₚ-COO)ₓR₂ dans laquelle x représente 1, 2 ou 3, R₁ est H ou un radical hydrocarboné comprenant de 4 à 11 atomes de carbone et le cas échéant une fonction hydroxyle, R₂ est H ou un radical alkyle comportant de 2 à 4 atomes de carbone et pouvant contenir un ou plusieurs hétérotatomes, et p un indice entier compris entre 2 et 11 , comportant une étape réactionnelle d'ammoniation conduisant à la transformation de la fonction carbonyle en fonction nitrile **caractérisé en ce que** :
- dans un premier stade on transforme l'acide/ester gras insaturé en un nitrile ω-insaturé de formule CH₂=CH-(CH₂)ₚ-CN en deux étapes successives, d'ordre indifférent, éthénolyse et ammoniation puis,
- dans un deuxième stade on transforme ce nitrile ω-insaturé en un nitrile ester de formule R₃OOC- CH=CH-(CH₂)ₚ-CN dans laquelle R₃ est n-butyle, par une réaction de métathèse croisée du nitrile w-insaturé avec un acrylate de formule CH₂=CH-COOR₃ avec un catalyseur de formule (I), 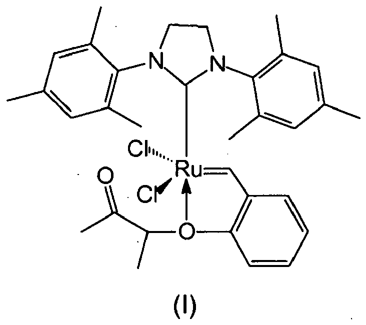 puis,
- dans un troisième stade on hydrogène le nitrile ester en ω-aminoacide(ester) de formule R₃OOC-(CH₂)_{q}-CH₂NH₂.

2. Procédé selon la revendication 1 **caractérisé en ce que** lors du premier stade on effectue tout d'abord l'éthénolyse de l'acide (ester) pour la faire suivre par l'ammoniation de l'acide ω-alcénoïque.

3. Procédé selon la revendication 1 **caractérisé en ce que** lors du premier stade on effectue tout d'abord l'ammoniation de l'acide (ester) pour la faire suivre par l'éthénolyse du nitrile de l'acide gras de départ.

4. Procédé selon la revendication 1 **caractérisé en ce que** lors du premier stade on effectue tout d'abord la pyrolyse de l'acide (ester) gras hydroxylé pour la faire suivre par l'ammoniation de l'acide(ester) ω-alcénoïque issu de la pyrolyse.

5. Procédé selon la revendication 1 **caractérisé en ce que** lors du premier stade on effectue l'ammoniation de l'acide (ester) sans procéder à la réaction d'éthénolyse.

## Patentansprüche

1. Verfahren zur Synthese einer ω-Aminosäure bzw.
eines ω-Aminosäureesters der Formel R₃OOC- (CH₂)_{q}-CH₂NH₂, worin R₃ für H oder einen n-Butylrest steht und q für eine ganze Zahl zwischen 2 und 13 steht, aus einer einfach ungesättigten Fettsäure bzw. einem einfach ungesättigten Fettsäureester der Formel (R₁-CH=CH-(CH₂)ₚ-COO)ₓR₂, worin x für 1, 2 oder 3 steht, R₁ für H oder einen Kohlenwasserstoffrest mit 4 bis 11 Kohlenstoffatomen und gegebenenfalls einer Hydroxylfunktion steht, R₂ für H oder einen Alkylrest mit 2 bis 4 Kohlenstoffatomen, der ein oder mehrere Heteroatome enthalten kann, steht und p für eine ganze Zahl zwischen 2 und 11 steht, umfassend einen Ammonierungsreaktionsschritt, der zur Umwandlung der Carbonylfunktion in eine Nitrilfunktion führt, **dadurch gekennzeichnet, dass** man:
- in einer ersten Stufe die ungesättigte Fettsäure bzw. den ungesättigten Fettsäureester in zwei aufeinanderfolgenden Ethenolyse- und Ammonierungsschritten in beliebiger Reihenfolge in ein ω-ungesättigtes Nitril der Formel CH₂=CH-(CH₂)ₚ-CN umwandelt, dann
- in einer zweiten Stufe dieses ω-ungesättigte Nitril durch eine Kreuzmetathesereaktion des ω-ungesättigten Nitrils mit einem Acrylat der Formel CH₂=CH-COOR₃ mit einem Katalysator der Formel (I) 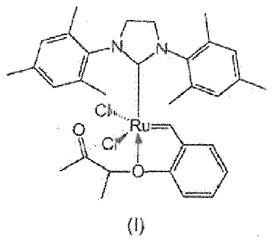 in einen Nitrilester der Formel R₃OOC-CH=CH-(CH₂)ₚ-CN, worin R₃ für n-Butyl steht, umwandelt dann
- in einer dritten Stufe den Nitrilester zu der ω-Aminosäure bzw. zu dem ω-Aminosäureester der Formel R₃OOC-(CH₂)_{q}-CH₂NH₂ hydriert.

2. Verfahren nach Anspruch 1, **dadurch gekennzeichnet, dass** man in der ersten Stufe zunächst die Ethenolyse der Säure bzw. des Esters durchführt und darauf die Ammonierung der ω-Alkensäure folgen lässt.

3. Verfahren nach Anspruch 1, **dadurch gekennzeichnet, dass** man in der ersten Stufe zunächst die Ammonierung der Säure bzw. des Esters durchführt und darauf die Ethenolyse des Nitrils der Ausgangsfettsäure folgen lässt.

4. Verfahren nach Anspruch 1, **dadurch gekennzeichnet, dass** man in der ersten Stufe zunächst die hydroxylierte Fettsäure bzw. den hydroxylierten Fettsäureester pyrolysiert und darauf die Ammonierung der aus der Pyrolyse stammenden ω-Alkensäure bzw. des aus der Pyrolyse stammenden ω-Alkensäureesters folgen lässt.

5. Verfahren nach Anspruch 1, **dadurch gekennzeichnet, dass** man in der ersten Stufe die Ammonierung der Säure bzw. des Esters durchführt, ohne zur Ethenolysereaktion überzugehen.

## Claims

1. A process for synthesizing an ω-amino acid (ester) of formula HR₃OOC-(CH₂)_{q}-CH₂NH₂, in which HR₃ is H or an n-butyl radical and q is an integral index of between 2 and 13, from a monounsaturated fatty acid (ester) of formula (R₁-CH=CH-(CH₂)ₚ-COO)ₓR₂, in which x represents 1, 2 or 3, R₁ is H or a hydrocarbon radical comprising from 4 to 11 carbon atoms and, where appropriate, a hydroxyl function, R₂ is H or an alkyl radical comprising from 2 to 4 carbon atoms, and may contain one or more heteroatoms, and p is an integral index of between 2 and 11, comprising a reaction step of ammoniation, leading to the conversion of the carbonyl function to a nitrile function, **characterized in that**:
- in a first stage, the unsaturated fatty acid/ester is converted into an ω-unsaturated nitrile of formula CH₂=CH-(CH₂)ₚ-CN in two successive steps, in any order, ethenolysis and ammoniation, and then
- in a second stage, this ω-unsaturated nitrile is converted into an ester nitrile of formula R₃OOC-CH=CH-(CH₂)ₚ-CN, in which R₃ is n-butyl, by a cross metathesis reaction of the ω-unsaturated nitrile with an acrylate of formula CH₂=CH-COOR₃, with a catalyst of formula (I), 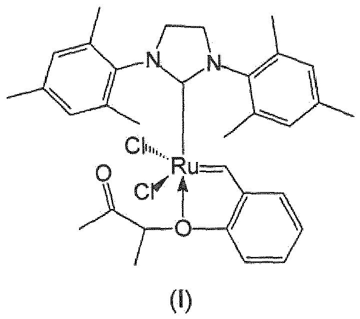 and then
- in a third stage, the ester nitrile is hydrogenated to ω-amino acid (ester) of formula R₃OOC-(CH₂)_{q}-CH₂NH₂.

2. The process as claimed in claim 1, **characterized in that** in the first stage the ethenolysis of the acid (ester) is carried out first, followed by the ammoniation of the ω-alkenoic acid.

3. The process as claimed in claim 1, **characterized in that** in the first stage, the ammoniation of the acid (ester) is carried out first, followed by the ethenolysis of the nitrile of the starting fatty acid.

4. The process as claimed in claim 1, **characterized in that** in the first stage the pyrolysis of the hydroxyl-containing fatty acid (ester) is carried out first of all, followed by the ammoniation of the ω-alkenoic acid (ester) obtained from the pyrolysis.

5. The process as claimed in claim 1, **characterized in that** in the first stage the ammoniation of the acid (ester) is carried out, without continuing to the ethenolysis reaction.
